# EUROPEAN PATENT APPLICATION

(11) **EP 1 431 312 A1**
(43) Date of publication of application: **23.06.2004**
(21) Application number: 01274393.6
(22) Date of filing: 26.10.2001
(51) Int. Cl.: C07K 17/08, C07H 21/00

(54) **METHOD FOR PRODUCTION AND A COMPOSITION FOR IMMOBILISING BIOLOGICAL MACROMOLECULES IN HYDROGELS AND THE USE OF SAID COMPOSITION FOR PRODUCING BIOCHIPS**

(30) Priority: 25.07.2001 RU 2001120905
(71) Applicant: INSTITUT MOLEKULYARNOI BIOLOGII IM. V.A.ENGELGARDTA ROSSIISKOI AKADEMII NAUK, Moscow, 117984 (RU)
(72) Inventor: MIRZABEKOV, Andrei Darievich, Moscow, 117420 (RU); RUBINA, Alla Jurievna, Moscow, 125493 (RU); PANKOV, Sergei Vasilievich, Samara, 443011 (RU); PEROV, Alexandr Nikolaevich, Moskovskaya obl. (RU); CHUPEEVA, Valentina Vladimirovna, Moscow, 121433 (RU)
(74) Representative: HOFFMANN - EITLE
(86) International application number: PCT/RU2001/000445
(87) International publication number: WO 2003/010203

(57) **Abstract**

The present invention refers to the field of molecular biology and bio-organic chemistry and deals with compositions for immobilization of modified oligonucleotides, proteins, nucleic acids or any other molecules bearing the unsaturated groups in a hydrogel, in manufacturing microchips by method of photo-initiated copolymerization. The invention also refers to the technology for manufacturing microchips and to performing a polymerase chain reaction (PCR) over a chip having an application in molecular biology for sequencing and mapping of DNA, detection of mutations, and in a whole lot of medical applications.

## Description

### Field of Invention

The invention refers to the field of molecular biology and bioorganic chemistry and deals with a composition for in hydrogel immobilization of modified oligonucleotides, proteins, nucleic acids, or any other molecules bearing the unsaturated groups in preparing biochips by method of photo-initiated copolymerization. The invention also refers to the technology for manufacturing microchips and to performing a polymerase chain reaction (PCR) over a chip having an application in molecular biology for sequencing and mapping of DNA, detection of mutations, and in a whole lot of medical applications.

### Background of Invention

There are known in the art publications on immobilization of modified oligonucleotides and proteins in a acrylamide gel in manufacturing biochips by method of copolymerization [1, 2].
[1] F. N. Rehman, M. Audeh, E. S. Abrams, P. W. Hammond, M. Kenney, and T. C. Boles, Nucleic Acids Research, 1999, V.27, Nº 15, P. 649-655.
[2] A. V. Vasiliskov, E. N. Timofeev, S. A. Surzhikov, A. L. Drobyshev, V. V. Shick, and A. D. Mirzabekov, BioTechniques, 1999, V.27, P. 592-606.

Conventionally, the compositions being used in publications cited for manufacturing the oligonucleotide and protein microchips may be subdivided into the following components:
- *Monomers making the basis of the gel being formed*
   Use is made of mixtures of acrylamide and *N,N'*-methylenbisacrylamide as a gel forming carrier having the total content T = 5%, C = 5% (19 : 1) [2], and T = 10%, C = 3.3% (29 : 1) [1].
- Modified oligonucleotides and proteins bearing the unsaturated groups
   There are proposed methods for synthesis of modified oligonucleotides and proteins bearing methacrylamide [1], acrylamide [2], and allyl [2] groups.
- Medium for performing the photo-initiated copolymerization

Use is made of water-glycerol solutions having ratios of water/glycerol equal to 25:75 [1] and 60:40 [2] to form a hydrogel.

The technology for immobilizing biological macromolecules in hydrogels finds application in practice particularly in manufacturing biological microchips (biochips) (Khrapko et al., US Patent No. 5552270; Ershov et al., US Patent No. 5770721). Presently, the biochips are considered as one of the most challenging analytical tools in the fields such as an investigation of DNA structure in molecular biology, a gene clinical diagnosis, monitoring pathogenic microorganisms etc. In these chips, the macromolecules playing a part of molecular probes are immobilized in hydrogel cells, which are fixed on a common substrate and form the regular structure (matrix).

There are known methods for manufacturing biochips based on hydrogels, in which a technological cycle consists of steps: (1) preparation of substrate, (2) forming a matrix of gel cells on the substrate, (3) application of the solution of biological macromolecules onto cells in line with biochip scheme predetermined, (4) chemical treatment of cells with aim to immobilize molecules of probes, (5) washing and drying of biochips obtained. To form matrix of gel cells, it is provided the method of laser ablation of a special light-absorbing layer located under the continuous gel layer having a geometry being complement with respect to the predetermined geometry of cells' body (Ershov et al., US Patent No. 5770721), as well as the method of photo-polymerization through a mask (Guschin et al., Manual manufacturing of Oligonucleotide, DNA, and Protein Microchips, Analytical Biochemistry, 1997, Vol. 250, No. 2, pp. 203 - 211).

The methods stated are technically complicated, and uses are made of procedures not ensuring the necessary uniformity and reproducibility of gel cell properties and are hardly automatized.

There are also known methods for manufacturing biochips based on a gel in which steps of forming the cells' body and immobilizing molecules of probe are combined together by using a technique of photo- or chemical initiated copolymerization [1, 2]. The essence of these methods consists in that use is made of polymerization mixtures, which comprise, along with monomer and cross-linking agent, immobilized macromolecules provided with the unsaturated group, which enables an incorporation of these molecules into a polymer net of hydrogel.

According to a protocol for manufacturing biochips as disclosed in reference [1], chip cells are obtained by polymerization of the mixture droplets supported on a substrate using a micropipette.

According to a method [2] for manufacturing a chip, use is made of a special thin-layer (≈5 µm) chamber having a reaction volume bounded, on the one hand, with a substrate of future biochip and, on the other hand, with a window transparent for UV radiation. The biochip cells have been developed one after another by performing a cycle of the following operations: (1) filling a chamber with the mixture having a corresponding probe, (2) polymerization of the mixture by exposure of UV-beam focused into a square blemish of the necessary size in the centre of location of future cell, (3) washing the chamber before its filling with the next solution.

It is clear that versions of copolymerization technology considered are also of little use for automatic manufacturing biochips. As to immobilization chemistry, the methods also have several drawbacks:
1. In manufacturing the gels, the application of acrylamide and *N,N'*-methylenbisacrylamide only greatly restricts the range of hydrogels obtained by their structure and porosity.
2. According to a method of authors [1] for preparing the modified oligonucleotides, it allows preparing the 5'-end methacrylamide group only linked to the oligonucleotide molecule through a phosphamide linkage being labile in acids [3-5]:
   [3] N.N. Preobrazhenskaya, Russ. Chem. Rev., 1972,41, P.54.
   [4] Chanley J., Feageson E., J.Am.Chem.Soc., 1965, 87, P.3199
   [5] Clark V., Kirby G., J.Chem.Soc., 1957, P.1497.
3. The problem of lability in acids is overcome in [2], however, the allyl terminal group, as inserted in the oligonucleotide molecule according to this method, has a low affinity to acrylamide and bisacrylamide in a copolymerization reaction.
4. The immobilization of proteins is provided by two-stage method of modification enabling an insertion of the unsaturated moiety by NH₂-groups only.
   For performing chemical polymerization of microchip elements in reference [1], it is proposed a damp nitrogen atmosphere that decreases the efficiency of polymerization.
   For performing photo-initiated polymerization in reference [2], use is made of UV radiation with the wavelength of 254 nm that results in destruction of oligonucleotides [6].
   [6] Saito I. et al. Tetrahedron Lett., 1981, 22, P. 3265-3268.

### Summary of the Invention

The essence of invention consists in that oligonucleotides, proteins, nucleic acids, or any other biological macromolecules are modified with various unsaturated groups having a high affinity with the unsaturated monomer being the basis of hydrogel formed (such as acrylamide, methacrylamide, or any other similar monomer) that allows performing the effective immobilization of modified biological macromolecules in a gel straight in the process of its preparation. In particular, this immobilization procedure forms the basis of method for preparing biological microchips based on hydrogels, which method is a constituent of the present invention.

According to the present method of immobilization of biological macromolecules in hydrogels, for preparing thereof, use is made of composition of the following formula:

**K = A**^{**a**}**+B**^{**b**}**+C**^{**c**}**+D**^{**d**}**+E**^{**e**}

wherein
**K** is a composition;
**A** is acrylamide, methacrylamide, *N*-[tris(hydroxymethyl)methyl]acrylamide, or another monomer based on derivatives of acrylic and methacrylic acids;
**B** is *N,N'*-methylenbisacrylamide, *N,N*'-1,2-dihydroxyethylenebisacrylamide, polyethyleneglycol diacrylate, a mixture thereof or other symmetric or asymmetric cross-linking water soluble agent based on derivatives of acrylic and methacrylic acids;
**C** is a modified oligonucleotide, modified nucleic acid, protein or another molecule bearing unsaturated group;
**D** is glycerol, sucrose, *N,N-*dimethylformamide, dimethylsulfoxide, polyhydric alcohols (e.g., polyvinyl alcohol), or another water soluble high-boiling compound;
**E** is water;
**a,b,c,d,e** are percentages of any ingredient in the composition (for solids X = m/v×100% and for liquids X = v/v×100%).
3%≤**a+b**≤40%; 0.0001%≤**c**≤10%; 0%≤**d**≤90%; 5%≤**e**≤95%.

Along with gels of another application, the composition as indicated above is proposed to use for manufacturing oligonucleotide, protein, and DNA (RNA) microchips by method of photo-initiated polymerization using the technology as follows.

The present composition for manufacturing biological oligonucleotide, protein, and DNA (RNA) microchips will make use of the following ingredients:
- Monomers composing the basis of gel being formed
   As gel-forming monomers, there are proposed various combinations of acrylamide, methacrylamide, *N*-[tris(hydroxymethyl)methyl]acrylamide, *N,N'*-methylene bisacrylamide, *N,N'*-1,2-dihydroxyethylene bisacrylamide, or any other water soluble monomer based on derivatives of acrylic and methacrylic acids. In this case the total content of monomer and cross-linking agent in the composition ranges from 3 to 40% (3<T<40), and the monomer to cross-linking agent ratio being in the range of 97:3-60:40% (3<C<40).
   Hydrogels having a predetermined pore size are to be prepared by varying the combinations and ratios of monomers.
- Modified oligonucleotides, proteins, nucleic acids, which structures comprise fragments of unsaturated acids.

### Methods of modification of oligonucleotides

For manufacturing oligonucleotide microchips there are provided synthetic oligonucleotides of general formula: 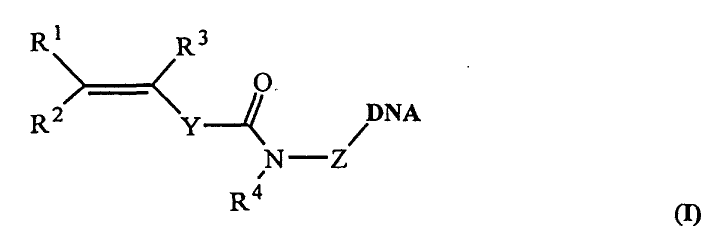 wherein
R¹, R², R³ are H, alkyl C₁-C₆, Ph, PhCH₂- ;
Z is (CH₂)ₙCH(CH₂OH)CH₂OX where n = 1-6; or (CH₂)ₙ-OX where n = 2-6;
X is a phosphodiester group binding an unsaturated moiety to 5'- and/or 3'-end of the oligonucleotide;
R⁴ represents H, (CH₂)ₙOH where n = 2-6;
Y is (*p*-C₆H₄)ₙ where n = 0-2;
being prepared by using conventional phosphoramidite solid-phase synthesis or acylation of synthetic, amino-linker comprising oligonucleotide with activated esters of unsaturated acid in post-automatic conditions.
- In automatic conditions, the insertion of unsaturated compounds into synthetic oligonucleotide is performed by using corresponding phosphoramidite. In the general case of modifying oligonucleotides, there are provided phosphoramidites of formula: 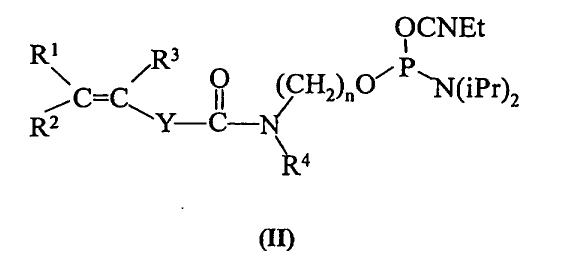
wherein
R¹, R² are H, alkyl C₁-C₆, Ph, PhCH₂-, where n = 2-6;
R³ is alkyl C₁-C₆;
R⁴ represents H, (CH₂)ₙODMT where n = 2-6;
Y is (p-C₆H₄)ₙ where n = 0-2.

### Example 1 represents a procedure for synthesis of 2-(N-methacrylaminoethyl)-N,N'-diisopropyl-2-cyanoethyl phosphoramidite.

For inserting unsaturated acid at 3'-end of oligonucleotides under conditions of automatic synthesis of oligonucleotides, there are provided a carrier porous glass (CPG) of the following structure: 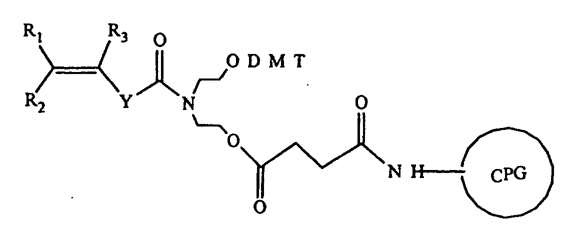 wherein:
R¹, R² are H, alkyl C₁-C₆, Ph, PhCH₂-;
R³ is alkyl C₁-C₆;
Y is (*p*-C₆H₄)ₙ, where n = 0-2,

Use is made of this modified CPG in conventional conditions of automatic synthesis of oligonucleotides.

### Example 2 sets forth a procedure for synthesis of methacrylamide-CPG.

- In-post-automatic conditions, the insertion of unsaturated fragment into oligonucleotide is performed by using reaction of 4-nitrophenyl ester of corresponding acid with the amino-linker comprising, synthetic oligonucleotide at 5'- and/or 3'-end. The 4-nitrophenyl esters of unsaturated acids are prepared by reaction of corresponding acids with 4-nitrophenol in the presence of dicyclohexyl carbodiimide or acid anhydrides (including chloranhydrides) in the presence of bases.

### Example 3 represents the general acylation procedure for synthetic oligonucleotides having unsaturated groups.

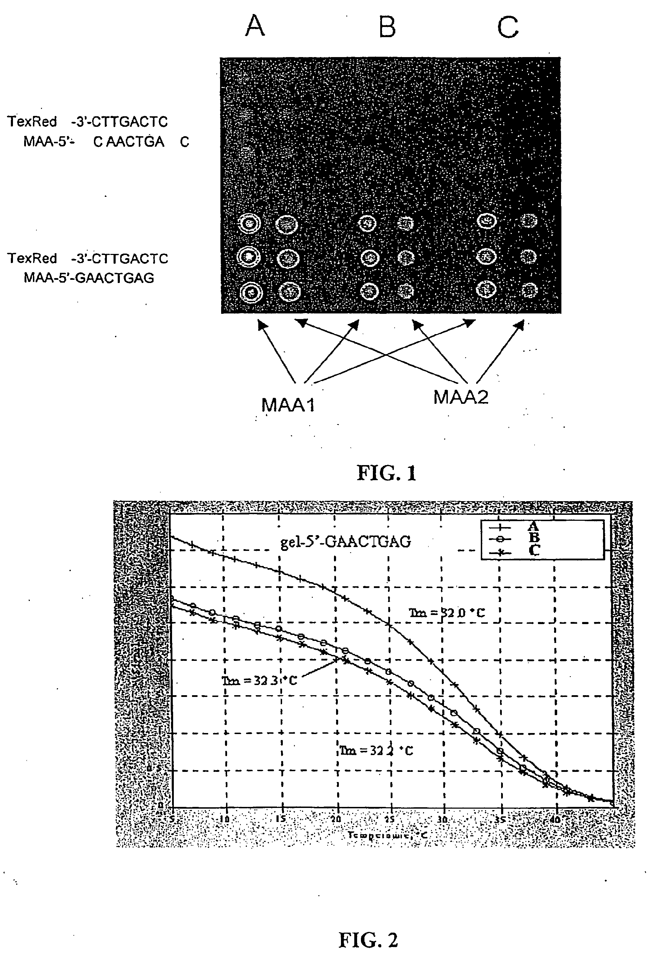

Fig. 1 represents results of hybridization over a oligonucleotide microchip wherein the oligonucleotide with 5'-terminal methacrylamide group is prepared under conventional conditions of automatic synthesis of oligonucleotides using phosphoramidites of formula (II).

### Methods of modification of DNA

For manufacturing DNA microchips there are provided three methods for modifying DNA fragments to insert unsaturated group in the molecule structure:
- Utilization of synthetic primer bearing a terminal unsaturated group in PCR-amplification.

This method allows preparing a double-stranded or one-stranded DNA fragment of the following structure: 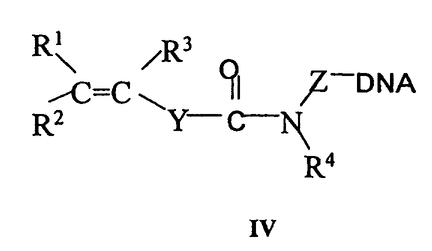 wherein:
R¹, R², R³ are H, alkyl C₁-C₆, Ph, PhCH₂- ;
Z is (CH₂)ₙCH(CH₂OH)CH₂OX where n = 1-6; or -(CH₂)ₙ-OX where n = 2-6;
X is a phosphodiester group binding an unsaturated moiety to the end fragment of the DNA;
R⁴ represents H, (CH₂)ₙOH where n = 2-6;
Y is (p-C₆H₄)ₙ where n = 0-2;
- Direct acylation of DNA fragments with unsaturated acid anhydride.

Utilization of unsaturated acid anhydrides for acylation of the DNA allows preparing modified DNA fragments of the following structure:

**5'-X-O-DNA-3'-O-Z** **(V)**

wherein
X is H, H₂PO₃ -CO-Y-CR³=CR²R¹;
Z represents H, H₂PO₃, -CO-Y-CR³=CR²R¹
R¹, R², R³ are H, alkyl C₁-C₆, Ph, PhCH₂- ;
Y represents (*p*-C₆H₄)ₙ where n = 0-2.

It is managed to do the direct acylation of DNA fragments not affecting the exocyclic amino groups of bases [7].
[7] Stuart A., Khorana H.G., J. Biol. Chem., 1964,239, P. 3885-3892.

### Example 4 sets forth a procedure for O-acylation of DNA fragments with anhydrides of unsaturated acids.

- Amination of DNA followed by acylation with activated esters of unsaturated acids. The DNA fragment aminated by procedure [8] is acylated with activated esters of unsaturated acids in water organic medium.

[8] Proudnikov D, Timofeev E, Mirzabekov A., Anal Biochem. 1998, 15; 259(1), P. 34-41.

The modified DNA fragments obtained have of the following structure: 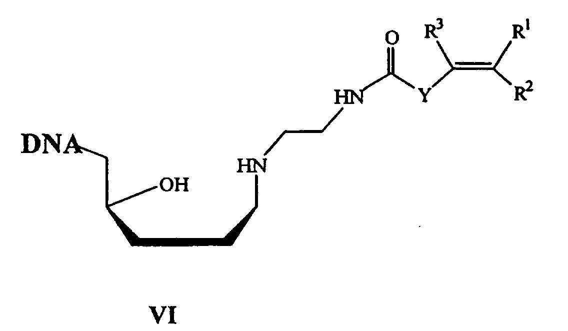 wherein:
R¹, R², R³ are H, alkyl C₁-C₆, Ph, PhCH₂- ;
Y is (p-C₆H₄)ₙ where n = 0-2.

### Example 5 sets forth a procedure for amination of DNA followed by acylation with activated esters of unsaturated acids.

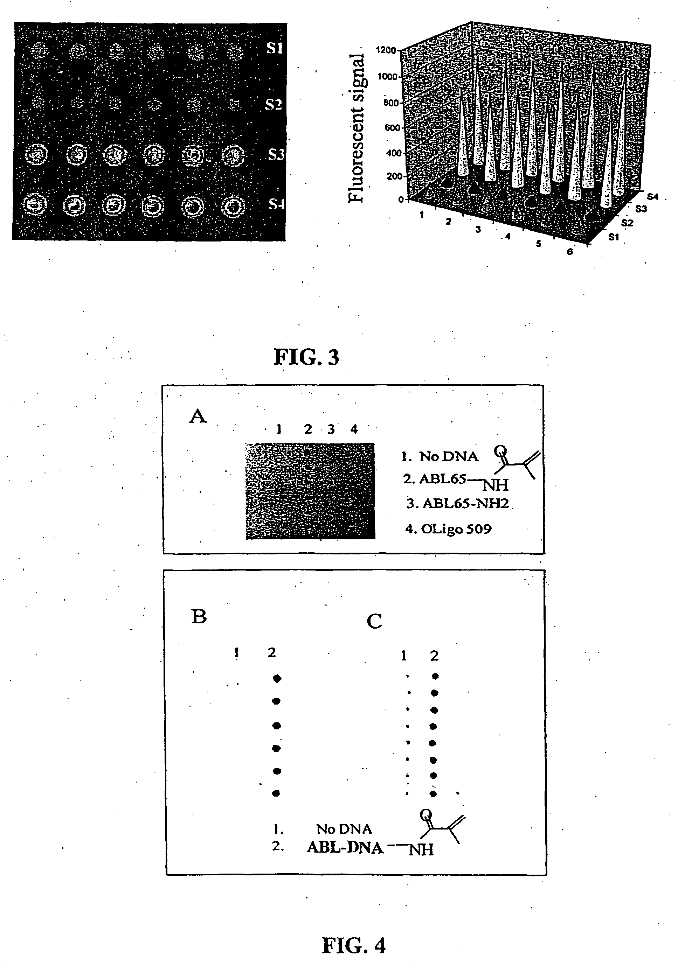

Fig. 4 shows the results of hybridization over microchips with the immobilized DNA fragments.

### Methods of modification ofproteins

### Modification of proteins is performed by three methods:

1. Acylation of proteins at amino groups is performed with activated esters of acids of the following structure: 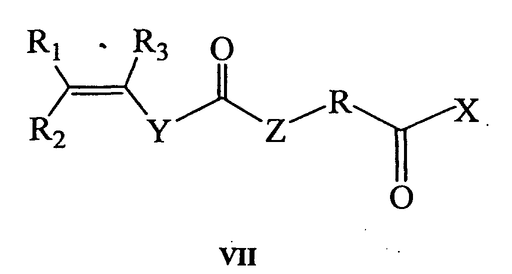 wherein:
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, where n = 1-20;
X is N-hydroxysuccinimidyl-, *p*-nitrophenyloxy-, pentafluoro phenyloxy-, or any other readily leaving acceptor group;
Z is NH, O, CH₂, S.

Modified proteins have the following structure: 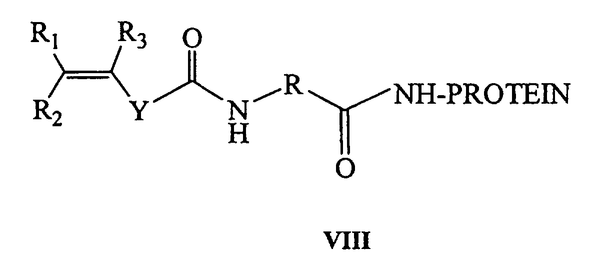 wherein
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, n = 1-20;
Z is NH, O, CH₂, S.

### Example 6 sets forth a procedure for modification of proteins at amino groups by example of Barnase protein.

Fig. 3 shows the results of hybridization over microchips with the immobilized Bamase protein.

2. Alkylation of protein's amino- or sulfhydryl groups with derivatives of α,β-unsaturated carbonyl compounds and α-halocarboxylic acids is performed by reagents of the following structure: 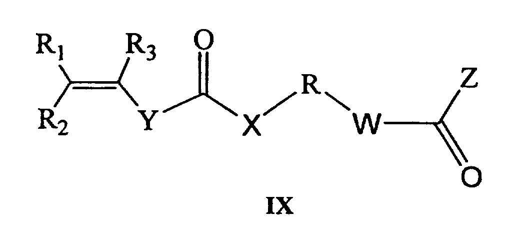 wherein
R¹, R², R³ are H, alkyl C₁-C₆, Ph, PhCH₂-;
Y is (*p*-C₆H₄)ₙ, where n = 0-2;
R is (CH₂)ₙ, (CH₂O)ₙ, n = 1-20;
X is NH, O, S, CH₂;
W is NH, O, CH₂;
Z is a halomethyl, vinyl, succinimidyl, or any other fragment comprising an active multiple bond.

Modified proteins have the following structure: 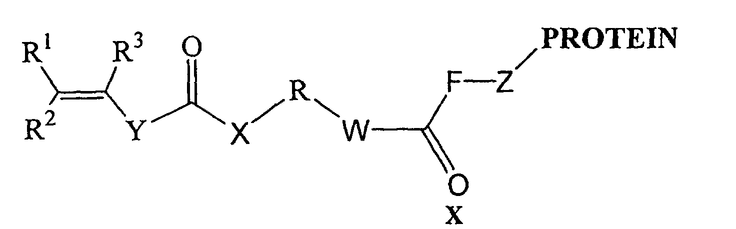 wherein
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, n = 1-20;
X is NH, O, S, CH₂;
W is NH, O, CH₂;
F is (CH₂)ₙ, n=1, 2;
Z is NH, S.

### Example 7 sets forth a procedure for alkylation of aspartate-aminotransferase from cytosol of hen's liver with 2-acryloyloxyethyl methacrylate.

3. A treatment of a His-6 recombinant protein with N-methacryoyl nitrilotriacetic acid in the presence of Ni(II) salts results in the formation of complex of the following structure:

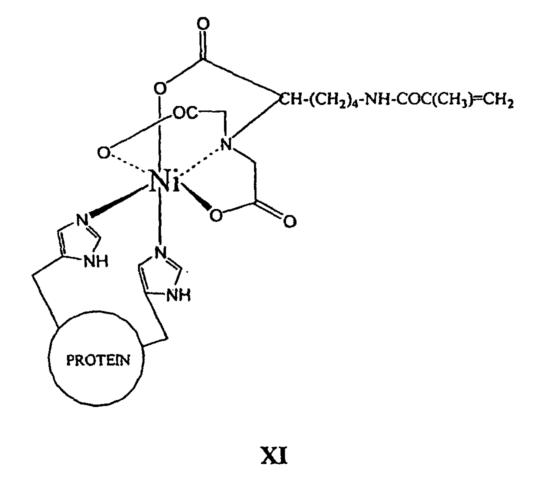

N-Methacryoyl nitrilotriacetic acid has the following structure:

**CH**_{**2**}**=C(Me)-CO-NH-R-CH(COOH)-N(CH**_{**2**}**COOH)**_{**2**}**,** **XII**

wherein R represents (CH₂)ₙ, (CH₂CH₂O)ₙ, n = 0-20.

**Fig. 4** represents results of hybridization over the protein microchip.

### • Medium for performing a photo-initiated polymerization.

As a medium in forming a gel, there are provided water-glycerol solutions, water-polyvinyl alcohol solutions, water-sucrose, solutions of *N,N-*dimethylformamide, dimethylsulfoxide and other water soluble high-boiling compounds. The content of water in gel-forming compositions ranges from 5% to 90% (v/v) and that of water soluble high-boiling compound ranges from 5% to 95% (m/v; v/v).

Depending on the water to high-boiling water soluble component ratio, there are obtained gel compositions of various viscosities that allow varying a size of gel elements of microchip under a pin robot diameter fixed.

### • Monomer being covalently bonded to the glass surface

For modifying a glass surface with a purpose of covalent bonding chip elements to the surface, there are provided the following reagents: 3-trimethoxysilylpropyl methacrylate, 3-trimethoxysilylpropyl methacrylamide, 3-trimethoxysilylpropyl acrylamide, 3-glicidyloxypropyl trimethoxysilane.

Such a range of modification agents allows preparing biochips which may be used under the broad ranges of pH (from 2 to 12) and temperatures from -10°C to +100°C.

Various combinations of composition ingredients allow preparing biochips, which elements' porosity may change over a broad range that makes it possible to use these biochips in many applications, particularly for performing a polymerase chain reaction (PCR), as well as to investigate an interaction in systems oligonucleotide-oligonucleotide, DNA-oligonucleotide, protein-protein, protein-DNA, etc.

The technology for manufacturing microchips by using a method of photo-initiated polymerization of the according to the present invention comprises the following aspects:
(1) method of preparation of compositions and substrates for manufacturing a microchip,
(2) method of application of microchip elements,
(3) method of leveling the water content in microchip elements,
(4) method of initiating a polymerization,
(5) method of washing microchip obtained,
(6) method of storing the compositions.

On preparation of compositions for manufacturing a chip, all components are carefully mixed to form a homogeneous solution, which is evacuated (P = 12 mm Hg) and transferred into plates for micro titration that will be further used as a source of solutions on application onto substrate (substrates) by using an automatic device (robot) which is equipped with one or more micro dispensers.

Method for manufacturing biochips is further disclosed in the case of using standard preparative glasses as substrates. However, it is clear that this does not restrict the field of application of methods for immobilizing macromolecules and manufacturing biochips because of techniques as follows may be readily adapted to substrates of another types, e.g. made of quartz, oxidized silicon, etc.

The preparation of glass to apply a composition for polymerization comprises a cleaning step by a sequential treatment with a concentrated alkali, acid, and a step of chemical modification by using solutions of one of 3-trimethoxysilylpropyl methacrylate, 3-trimethoxysilylpropyl methacrylamide, 3-trimethoxysilylpropyl acrylamide, 3-glicidyloxypropyl trimethoxysilane in organic solvents.

In biochip, the structure of the cells' body is formed by application of the regular body of micro droplets of polymerization composition onto the substrate prepared. In the general case, any micro droplet comprises macromolecules of special type. Depending on the viscosity and other properties of the polymerization compositions, and on the desired size of biochip cells, for application, use is made of robots having microdispensers of various types. Particularly, in case of application of viscous solutions having the content of glycerol over 40%, use are made of robot equipped with microdispensers of a rod (pin) type, such as robot "417 Arrayer" made in Affymetrix Co. (USA). In this case, depending on the rod diameter of dispenser, there are obtained droplets (and consequently gel cells) of various size.

Because of sequential character of applying droplets on formation the body, a discrepancy of duration of their contact with ambient air results in the different composition of droplets to the completion time of the application, mainly due to processes of water adsorption/desorption. This phenomenon results in heterogeneity of cells' size in the body formed. For solving the problem, following the application, biochips are placed into the sealed container, which contains a polymerization mixture having the composition similar to the application mixture, but free of biological macromolecules. This mixture volume should exceed the aggregate volume of applied micro droplets at least two times. An incubation period of biochips in the container is at least 2 h. The biochips are further saturated with dried inert gas (nitrogen, argon, carbon dioxide, etc.).

The polymerization process is initiated with UV-radiation (λ ≥ 312 nm) in the inert gas atmosphere. The biochips obtained are washed in distilled water at 60°C for 5 h, and air-dried at 25°C.

The compositions prepared may be stored at temperature -21°C at least for 1 year.

### Polymerase chain reaction (PCR) over a chip

A manufacturing oligonucleotide biochips is one of the most important application of the technology disclosed (immobilizing oligonucleotides in a gel by method of copolymerization). Uses are made of biochips prepared both in hybridization experiments and in performing more complex processes, such as polymerase chain reaction (PCR) over a chip. Following are set forth the demonstration examples of utilization of PCR over a chip (both in cases where PCR-solution covers gel cells of chip and where PCR proceeds only inside of gel cells being isolated each other and surrounded with a mineral oil).

Reaction PCR over a chip is also applied for a total characterization of copolymerized gel elements of chip having various compositions and for selection of the optimal method of their application. The main factors affecting an efficiency of PCR-analysis over the chip are a facile diffusion of DNA fragments measuring about 140 nucleotides (gel porosity), stability of cells over a broad range of pulsating temperatures, and availability and efficiency of immobilized oligonucleotide participation in hybridization and allele specific polymerase reaction to elongate its chain.

Example 8 sets forth a procedure for carrying out the PCR over a chip.

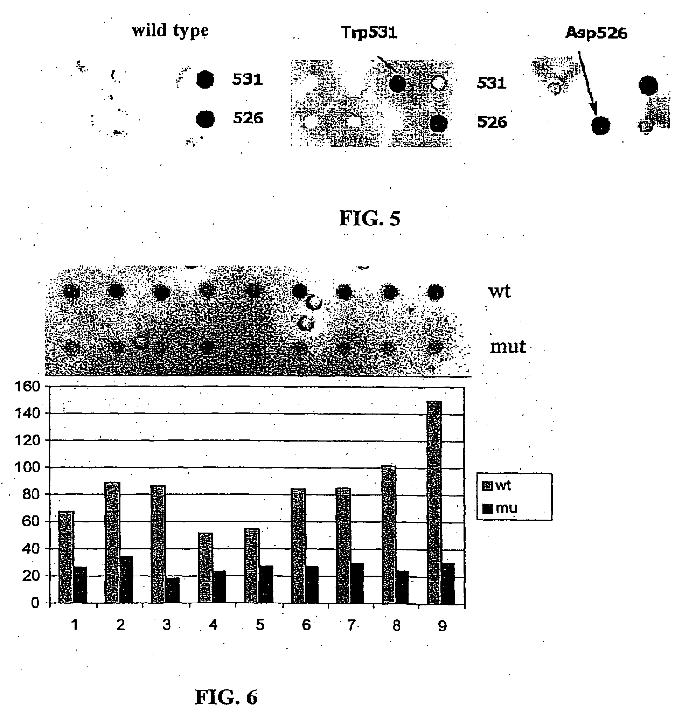

Figs. 5, 6 illustrate the results of PCR-analysis over the chip.

The subject matter of invention is further disclosed by the specific Examples not to be construed as a restriction of the following claims.

### Examples

### Example 1. Synthesis of 2-(N-methacrylaminoethyl)-N,N'-diisopropyl-2-cyanoethyl phosphoramidite

**Methacrylic acid 4-nitrophenyl ester**. To a solution of methacrylic acid (1.015 g, 11.79 mmole) and 4-nitrophenol (1.804 g, 12.98 mmole) in ethylacetate (14 mL) at 0°C a solution of 1,3-dicyclohexyl carbodiimide (2.68 g, 12.98 mmole) in ethylacetate (7 mL) is added and the mixture is strirred for 15 h at room temperature. A suspension is filtered through a glass filter. The filtrate is evaporated in vacuum reducing a volume to 10 ml and cooled down to minus 50°C. The precipitate formed is readily filtered through a glass filter and dried in vacuum. Yield is of 2.12 g (87%), Rf = 0.86 (acetone/petroleum ether = 3:2), melting point (Mp) 94-95°C.
¹H NMR (DMSO-d₆) δ: 2.10 (s, 3H, CH₃), 5.95 (s, 1H, CH₂=), 6.33 (s, 1H, CH₂=), 7.50 (appar. d, J= 9.0 Hz, 2H, C₆H₄), 8.31 (appar. d, J= 9.15 Hz, 2H, C₆H₄).
¹³C NMR (DMSO-d₆) δ: 18.85 (CH₃), 124.16, 126.20, 145.98, 156.43 (C₆H₄), 129.67, 135.72 (CH₂=C), 165.51 (-COO-).
Calculated for C₁₀H₉NO₄: C, 57.97; H, 4.35%;
Found: C, 57.99; H, 4.50%.

***N*-(2-Hydroxyethyl) methacrylamide.** To a solution of methacrylic acid 4-nitrophenyl ester (5.00 g, 24.15 mmole) in CH₃CN (40 mL) at 0°C a solution of ethanolamine (2.94 g, 48.30 mmole) in CH₃CN (40 mL) is added and the mixture is stirred for 15 h at room temperature. A suspension is filtered through a glass filter. The filtrate is evaporated in vacuum. The residuum is purified using silica column flash chromatography (acetone/petroleum ether = 3:2). Yield is of 2.804 g (90%), Rf = 0.41 (acetone: petroleum ether = 3:2), a straw oil.
¹H NMR (DMSO-d₆) δ: 1.85 (s, 3H, CH₃), 3.17 (appar. q, *J*= 5.92 Hz, 2H, NH-CH₂), 3.42 (appar. q, *J*= 5.91Hz, 2H, -CH₂O-), 4.55 (t, *J*= 5.29Hz, 1H, OH), 5.26 (appar. s, 1H, -CH=), 5.65 (appar. s, 1H, -CH=), 7.75 (br s, 1H, -NH-).
¹³C NMR (DMSO-d₆) δ: 19.61 (CH₃), 42.74 (-NH-CH₂), 60.75 (-CH₂O-), 119.67, 140.85 (CH₂=C), 168.34 (-COO-).
Calculated for C₆H₁₁NO₂: C, 55.81; H, 8.53%;
Found: C, 55.87; H, 8.64%.

### 2-(N-methacrylaminoethyl)-N,N'-diisopropyl-2-cyanoethyl phosphoramidite

To a solution of *N*-(2-hydroxyethyl) methacrylamide (0.1 mmole) in CH₃CN (0.25 mL) and 2-cyanoethyl-N,N'-tetraisopropyl phosphoramidite (0.095 mmole) a solution of tetrazol (0.095 mmole) in CH₃CN (0.25 mL) is added. The mixture is stirred for 15 h at room temperature. The precipitate formed is readily filtered through a glass filter and filtrate is used in solid-phase synthesis of oligonucleotides without purification.

### Example 2. Synthesis of methacrylamido-CPG carrier

Succinic anhydride (0.060 g, 0.3 mmole) and 4-(*N,N-*dimethylamino)pyridine (DMAP, 0.037 g, 0.3 mmole) are added to solution of *N*-(2-hydroxyethyl)-*N*-{2-[di-(4-methoxyphenyl)-phenylmethoxy]ethyl}methacrylamide (0.143 g, 0.3 mmole) in dry pyridine (3 mL). The mixture allows standing for 12 h at room temperature. A saturated solution of NaHCO₃ (2 mL) is added to solution. A solvent is evaporated in vacuum. Residuum is dissolved in ethyl acetate (5 mL) and organic layer is sequentially washed with saturated solution of NaHCO₃, several times with water, dried with anhydrous Na₂SO₄ and filtered. A solvent is evaporated to obtain a colorless syrup. Residuum is dissolved in dry pyridine (30 mL). To solution is added 2,2'-dipyridyl disulfide (0.060 g, 0.3 mmole), triphenyl phosphine (0.079 g, 0.3 mmole) and the LCA CPG (1.00 g). A suspension is concentrated in vacuum to a volume of 15 mL and held for 24 h at room temperature under periodically shaking. The suspension is filtered through a glass filter and residuum is sequentially washed with dry pyridine several times. To residuum washed the "capping" solution (acetic anhydride/DMAP/2,6-lutidine/acetonitrile) (10 mL) is added and the mixture allows standing for 5 min. Residuum is sequentially washed with dry pyridine several times, acetone, and diethyl ether. The modified carrier obtained (methacrylamido-CPG) has a concentration of unsaturated groups of 33.7 µmole/g (by test to a trityl group).

### Example 3. General procedure of acylation on synthesis of oligonucleotides with unsaturated groups

Acylation procedure for oligonucleotides having end-group of aliphatic amine with methacrylic acid 4-nitrophenyl ester is disclosed as an example.

To solution of modified oligonucleotide (32.1 nanomole) comprising unprotected amine-group at 5'- end (or 3'-) in a borate buffer (50 µL, pH 9.53) the solution of methacrylic acid 4-nitrophenyl ester (3.2 micromole) in DMF (110 µL) is added.

Homogeneous solution is allowed to stand overnight at 40°C. Nucleotides are separated by gel-filtration over Sephadex G25 and purified by using reverse phase HPLC. Yield is of >72%.

### Example 4. O-Acylation of DNA fragments with unsaturated acid anhydrides.

A solution of methacrylic acid anhydride (15 µL) in DMF (100 µL) is added to a fragment of DNA (10 picomole) dissolved in H₂O (50 µL) and cooled down to 5°C, then a solution of sodium hydroxide is added by small portions, and the pH-value being maintained at 7.0. To reaction mixture is added water (500 µL) 15 min later, and the volume of reaction medium is brought to 50 µL by sequential addition of some portions of anhydrous butanol with following extraction of the aqueous butanol. The modified DNA fragment is precipitated with a solution of lithium perchlorate in acetone (2%, 1000 µL) at minus 21°C. The precipitate is centrifuged and washed with acetone.

### Example 5. Amination of DNA followed by acylation with activated ester of unsaturated acid

Formic acid (80%, 20 µL) is added to the solution of oligonucleotide or DNA fragment (39.3 nmole) in H₂O (15 µL) and held at room temperature for 1 h. The oligonucleotide matter is further precipitated with a solution of lithium perchlorate in acetone (2%, 1000 µL), washed with acetone, and dried. The solution (0.5M) of ethylenediamine hydrochloride (50 µL, pH 7.4) is added to the purine free DNA and held for 3 h, at 37°C. Then the makeup solution of sodium hydroboride in water (15 µL, 0.1 M) and 30 min later the solution of ethylenediamine hydrochloride (20%, 14.75 µL) is added. The oligonucleotide matter is precipitated with a solution of lithium perchlorate in acetone (2%, V=1000 µL), washed with acetone and dried. Residuum is dissolved in the borate buffer (50 µL, pH 9.3) and the solution of methacrylic acid *p*-nitrophenyl ester (0.001 g) in DMF (100 µL) is added thereto. The solution is held for 12 h at 35°C. The modified DNA fragment is purified by gel-filtration.

### Example 6. Modification of proteins at amine group

The procedure of the protein Bamase modification is set forth as an example. A solution of 6-methacryloylaminohexanoic acid, *N*-hydroxysuccinimide ester (20 µL) (C=0.1 mg/mL) in DMF is added to 100 µL of Barnase solution (*C*=1 mg/mL) in 0.01 M borate buffer (pH 8.3). A reaction mixture is strirred for 30 min at room temperature. The modified protein obtained is further purified of low molecular weight reaction products and of DMF over micro concentrators Nanosep 3K Omega, washing out the protein with a borate buffer (pH 8.3). In 100 µL of buffer solution, the protein purified has the final concentration of 0.9 Mr/mL defined by spectrophotometric analysis. The modified protein obtained comprises one inserted 6-methacryloylaminohexanoic group according to MALDI-MS analysis.

The variation of ratio protein/modifying agent allows manufacturing a protein of a various extent of modification.

### Example 7. Alkylation of proteins at SH groups

The procedure of alkylation of aspartate-aminotransferase from cytosol of hen's liver with 2-acryloyloxyethyl methacrylate is set forth as an example.

The solution of 2-acryloyloxyethyl methacrylate (0.012 g, 65.2 µmole) in DMF (3 µL) is added to the solution of aspartate-aminotransferase (9 µL, 3.33 mg/mL) in a buffer (25 mM HEPES, pH 8.0, 30 mM KCl, 2 mM MgCl₂) cooled down to 5°C. The homogeneous solution allows standing for 1 h and modified protein is further purified over membrane filters. The effectiveness of protein modification is controlled by an electrophoretic method. Modified protein is utilized for manufacturing protein microchips.

### Synthesis of 2-acryloyloxyethyl methacrylate

Acrylic acid chloride (0.744 g, 8.22 mmole) is added to the solution of 2-hydroxyethyl methacrylate (1.070 g, 8.22 mmole) and triethylamine (0.832 g, 8.22 mmole) in tetrahydrofurane (25 mL) under vigorous stirring. The mixture is strirred at room temperature for 1 h, a precipitate formed is filtered off, and filtrate is concentrated. Water (30 mL) is added to residuum obtained (oil) and the mixture is vigorously shaken. A water layer is separated, ethyl acetate (15 mL) is added, the organic layer is dried over sodium sulfate and is evaporated in vacuum. Yield is of 81%, oil.
¹H NMR (DMSO-d₆) δ: 1.87 (s, 3H, CH₃), 3.34 (m, 2H, O-CH₂), 3.37 (m, 2H, -CH₂-O), 5.68 (pseudo-s, 1H, CH₂=); 6.02 (pseudo-s, 1H, CH₂=); 5.96 (dd, J₁=10.26 Hz, J₂=1.87 Hz, 1H, CH₂,=); 6.33 (dd, J₁= 17.13 Hz, J₂= 1.55 Hz, 1H, CH₂=); 6.19 (dd, J₁= 17.13 Hz, J₂ =10.28 Hz, 1H, =CH-).
Calculated for C₉H₁₂O₄: C, 58.70; H, 6.52%; Found: C, 58.63; H, 6.64%.

### Example 8. Polymerase chain reaction (PCR) over a chip

Typical PCR-experiment *in situ* is performed over a chip in special micro-camera as described earlier (Strizhkov et al., 2000). Using the copolymerization method, in different cells of chip, oligonucleotides are immobilized that completely corresponds to a sequence of wild type, or oligonucleotides comprising an oligonucleotide substitute at 3'-end. Standard PCR-solution (67 mM Tris-HCl, pH 8.6; 2.5 mM MgCl₂; 16.6 mM (NH₄)₂SO₄; 0.001% Triton X-100; 1 mg/mL BSA; 0.24 mM each of dATP, dCTP, dGTP, and dTTP; 2.5 U Taq of DNA-polymerase in 30 µL) also contains about 10⁵ copies of the genomic DNA of *Mycobacterium tuberculosis,* as well as forward (F) primer (about 1 picomole) and labeled with Texas Red at 5'-end reverse (R) primer (about 10 picomole). Usually, there are performed 35 PCR-cycles: 40 s at 95°C, 60 s at 64°C, and 40 s at 72°C. Once the PCR have finished, the biochip is washed with solution of 0.1-0.3 M NaCl at 80°C consequently there remains only duplexes with elongated (due to reaction) primer over the chip, which may be detected by a fluorescent microscopy. Fig. 5 illustrates the result of one of these experiments.

### PCR inside of gel elements under mineral oil

Special experiment is performed to demonstrate an opportunity to realize the polymerase chain reaction (PCR) totally over a chip inside of individual gel element being obtained by copolymerization method. The option of PCR over a chip under mineral oil is utilized (Tillib et al., 2001). Use is initially made of the fragmented (200-300 nucleotides); denaturized genomic DNA of *M. tuberculosis* for hybridization with the corresponding oligonucleotides over a chip as described in the experiment above. Then, after washing the DNA not involved in hybridization, the chip is incubated with the standard PCR-solution (see above) for 30 min at 55°C. Water solution is substituted with a mineral oil and PCR is performed (30 cycles): 40 s at 72°C, 40 s at 95°C, and 60 s at 64°C. Subsequent to the final step of elongation (10 min at 72°C), the biochip is carefully washed first with chloroform, then with a solution of 0.1-0.3 M NaCl at 80°C, and is further examined by fluorescent microscopy.

Fig. 2 shows the result of such an experiment. One may see that allele specific PCR-reaction again proceeds quite efficiently in cells with a primer being entirely complementary to the primer of DNA used (wild type, wt, cf. cells with C4 primer). This reaction proceeds notably weaker in cells with a primer comprising an oligonucleotide substitute at 3'-end (mut, C5 primer) (Fig. 6).

## Claims

1. A method for preparing compositions for immobilization of biological macromolecules: oligonucleotides, proteins, DNA (RNA), or any other molecules bearing the unsaturated groups, **characterizing in that** said compositions have the following formula:
**K = A**^{**a**}**+B**^{**b**}**+C**^{**c**}**+D**^{**d**}**+E**^{**e**}
wherein
**A** is acrylamide, methacrylamide, *N*-[tris(hydroxymethyl)methyl]acrylamide, or any other monomer based on derivatives of acrylic and methacrylic acids;
**B** is *N,N'*-methylenebisacrylamide, *N,N'*-1,2-dihydroxyethylenebisacrylamide, polyethyleneglycol diacrylate, a mixture thereof or other cross-linking water soluble agent;
**C** is a modified oligonucleotide, modified nucleic acid, protein or other molecule bearing an unsaturated group;
**D** is glycerol, sucrose, *N,N-*dimethylformamide, dimethylsulfoxide, polyhydric alcohols (e.g., polyvinyl alcohol), or other water soluble high-boiling compound;
**E** is water;
**K** is a composition;
**a,b,c,d,e** are percentages (X) of each ingredient in the initial composition (for solids X = m/v×100% and for liquids X = v/v×100%);
3%≤**a+b**≤40%; 0.0001%≤**c**≤10%; 0%≤**d**≤90%; 5%≤**e**≤95%,
and provide a high extent of immobilization of biological macromolecules by using a copolymerization in hydrogels.

2. A method of claim 1 wherein monomers (**A, B**) are acrylamide, methacrylamide, *N*-[tris(hydroxymethyl)methyl]acrylamide, *N,N'*-methylenebisacrylamide, *N,N'-*(1,2-dihydroxyethylene)bisacrylamide, polyethyleneglycol diacrylate or other water soluble unsaturated compound.

3. A method of claim 1 wherein **C** is a modified oligonucleotides, nucleic acids, proteins or other molecules bearing unsaturated group.

4. A method of claim 1 wherein **D** is glycerol, polyvinyl alcohol, sucrose, or other water soluble high-boiling compound.

5. A method of claim 2 wherein the total content of monomer and cross-linking agent in the initial composition ranges from 3 to 40% (3<T<40), and the monomer to cross-linking agent ratio being in the range of 97:3-60:40% (3<C<40).

6. A method of claim 3 wherein 0.0001%≤**c**≤10%.

7. A method of claim 4 wherein 0%≤**d**≤90%.

8. A method of claim 1 wherein 5%≤**e**≤95%.

9. A method of claim 1 wherein polymeric hydrogel is obtained by copolymerization of mixtures of combinations from acrylamide, methacrylamide, *N*-[tris(hydroxymethyl)-methyl]acrylamide, *N,N'*-methylenebisacrylamide, *N,N'*-(1,2-dihydroxyethylene)-bisacrylamide, polyethyleneglycol diacrylate or other water soluble unsaturated compounds.

10. A method of claim 1 wherein hydrogels, having a predetermined pore size and improved thermal stability, are to be prepared by varying the combinations of composition ingredients.

11. A method of claim 3 wherein modified oligonucleotides have the following structure: 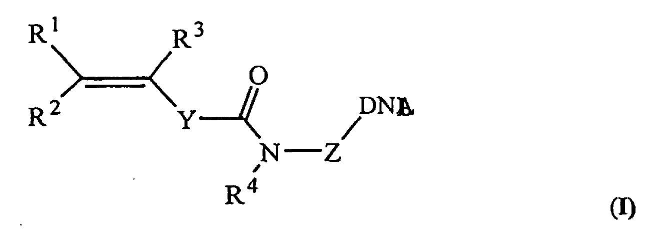 wherein
R¹, R², R³ represent H, alkyl C₁-C₆, Ph, PhCH₂- ;
Z is (CH₂)ₙCH(CH₂OH)CH₂OX where n = 1-6; or (CH₂)ₙ-OX where n = 2-6;
X is a phosphodiester group binding an unsaturated moiety to 5'- and/or 3'-end of the oligonucleotide;
R⁴ represents H, (CH₂)ₙOH where n = 2-6;
Y is (*p*-C₆H₄)ₙ where n = 0-2.

12. A method of claim 11 wherein a modification of oligonucleotides with unsaturated groups is performed in automatic conditions.

13. A method of claim 12 wherein a modification of oligonucleotides with unsaturated groups is performed by using corresponding phosphoramidite of general formula: 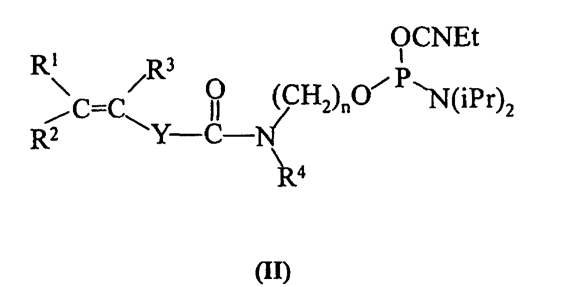 wherein
R¹, R² are H, alkyl C₁-C₆, Ph, PhCH₂-, where n = 2-6;
R³ is alkyl C₁-C₆;
R⁴ represents H, (CH₂)ₙ-ODMT where n = 2-6;
Y is (*p*-C₆H₄)ₙ where n = 0-2.

14. A method of claim 11 wherein a modification of oligonucleotides with unsaturated groups is performed in post-automatic conditions.

15. A method of claim 14 wherein a modification of oligonucleotides with unsaturated groups is performed by acylation of amino-linker comprising oligonucleotide with activated ester of unsaturated acid.

16. A method of claim 15 wherein an activated ester of unsaturated acid represents 4-nitrophenyl ester.

17. A method of claims 12 and 14 wherein a modification of oligonucleotides with unsaturated groups is performed at 5'-end of oligonucleotide.

18. A method of claims 12 and 14 wherein a modification of oligonucleotides with unsaturated groups is performed at 3'-end of oligonucleotide.

19. A method of claim 3 wherein modified DNA fragments are selected from formulas **IV, V,VI** 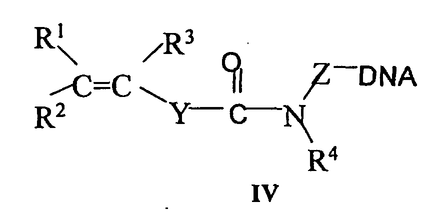 wherein:
R¹, R², R³ are H, alkyl C₁-C₆, Ph, PhCH₂- ;
X is a phosphodiester group binding an unsaturated moiety to 5'-end and/or 3'-end of oligonucleotide;
R⁴ represents H, (CH₂)ₙOH where n = 2-6;
Y is (p-C₆H₄)ₙ where n = 0-2;
or
**5'-X-O-DNA-3'-O-Z** **(V)**
wherein
X is H, H₂PO₃, -CO-Y-CR³=CR²R¹;
Z represents H, H₂PO₃, -CO-Y-CR³=CR²R¹
R¹, R², R³ are H, alkyl C₁-C₆, Ph, PhCH₂-;
Y represents (*p*-C₆H₄)ₙ where n = 0-2.
or 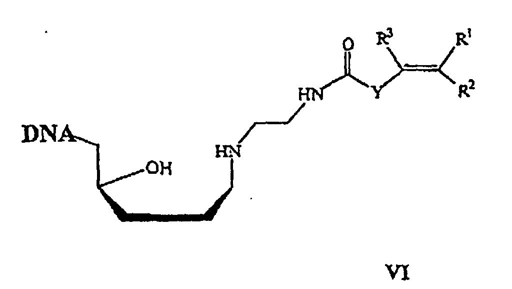 wherein:
R¹, R², R³ are H, alkyl C₁-C₆, Ph, PhCH₂- ;
Y is (p-C₆H₄)ₙ where n = 0-2.

20. A method of claim 19 wherein modified DNA fragments with formula **IV** are prepared by PCR-amplification using a synthetic primer bearing an unsaturated group at 5'-(3')-end.

21. A method of claim 19 wherein modified DNA fragments with formula **V** are prepared by direct acylation of DNA fragments with anhydrides of unsaturated acids

22. A method of claim 19 wherein modified DNA fragments with formula **VI** are prepared by reductive amination of the DNA followed by acylation with activated esters of unsaturated acids.

23. A method of claim 3 wherein modified proteins are selected from formulas **VIII, X, XI** 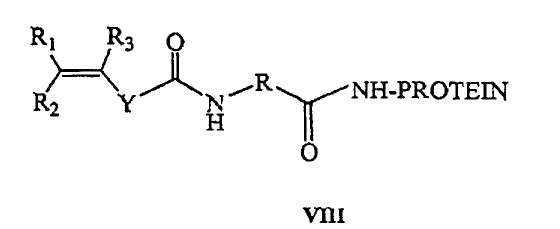 wherein
R¹, R², R³ are H, alkyl C₁-C₆, Ph, PhCH₂-;
Y is (*p*-C₆H₄)ₙ, where n = 0-2;
R is (CH₂)ₙ, (CH₂O)ₙ, n = 1-20;
Or 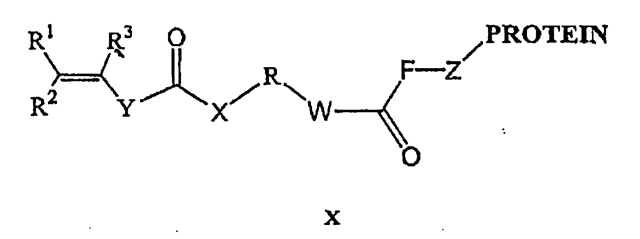 wherein
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, n = 1-20;
X is NH, O, S, CH₂;
W is NH, O, CH₂;
F is (CH₂)ₙ, n=1, 2;
Z is NH, S.

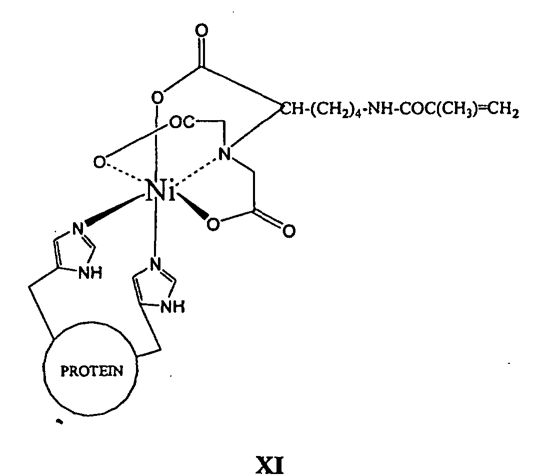

24. A method of claim 23 wherein modified proteins with formula VIII are prepared by acylation of amino groups with activated esters of corresponding unsaturated acids.

25. A method of claim 24 wherein activated esters have the following structure: 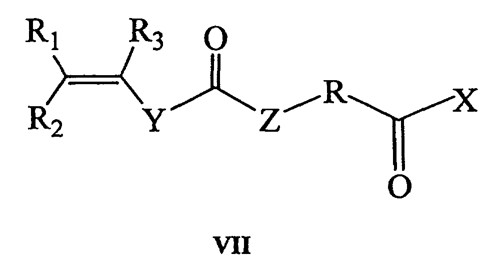 wherein:
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, where n = 1-20;
X is N-hydroxysuccinimidyl-, *p*-nitrophenyloxy-, pentafluoro phenyloxy-, or any other readily leaving acceptor group;
Z is NH, O, CH₂, S.

26. A method of claim 23 wherein modified proteins with formula X are prepared by alkylation of amino- or sulfhydryl groups with derivatives of α,β-unsaturated carbonyl compounds and α-halocarboxylic acids

27. A method of claim 23 wherein modified proteins with formula **XI** are prepared by treatment of a His-6 recombinant protein with N-methacryoyl nitrilotriacetic acid in the presence of Ni(II) salts

28. A method of claim 27 wherein N-methacryoyl nitrilotriacetic acid have the following structure:
**CH**_{**2**}**=C(Me)-CO-NH-R-CH(COOH)-N(CH**_{**2**}**COOH)**_{**2**}**, XII**
wherein R represents (CH₂)ₙ, (CH₂CH₂O)ₙ, n = 0-20.

29. A method of claim 26 wherein α,β-unsaturated carbonyl compounds have the following structure: 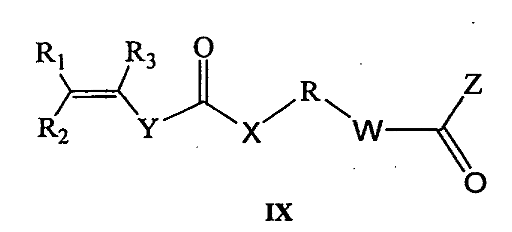 wherein
R¹, R², R³ are H, alkyl C₁-C₆, Ph, PhCH₂-;
Y is (*p*-C₆H₄)ₙ, where n = 0-2;
R is (CH₂)ₙ, (CH₂O)ₙ, n = 1-20;
X is NH, O, S, CH₂;
W is NH, O, CH₂;
Z is a halomethyl, vinyl, succinimidyl, or any other fragment comprising an active multiple bond.

30. A method of claim 1 wherein gel layer formed on a substrate is divided by empty spaces into several cells and each cell will comprise or not comprise macromolecules immobilized, and macromolecule being immobilized in various cells will have different nature and properties.

31. A method of claim 30 wherein said cells form the regular one- or two-dimensional structure (phase).

32. A method of claim 30 wherein an application of the polymerization mixture on substrate is carried out by using an automatic device (robot) equipped with one or more micro dispensers.

33. A method of claim 32 wherein use is made of micro dispensers of rod type.

34. A method of claim 32 wherein use is made of contactless micro dispensers of jet type.

35. A method of claim 32 wherein use is made of several micro dispensers forming a regular structure.

36. A method of claim 30 wherein before polymerization, one or more substrates including applied droplets of polymerization mixture are held in a sealed container which contains the similar polymerization mixture in amounts exceeding at least two times the aggregate volume of applied polymerization mixture.

37. A method of claim 30 wherein before and during polymerization, one or more substrates including applied droplets of polymerization mixture are placed in a sealed container under oxygen free inert atmosphere with a controlled humidity.

38. A method of claim 37 wherein said container is filled with one of the following gases: N₂, Ar, CO₂.

39. A method of claim 36 wherein gaseous medium is continuously or periodically restored in the container with substrates.

40. A composition for immobilization of biological macromolecules: oligonucleotides, proteins, DNA (RNA), or any other molecules bearing the unsaturated groups having the following formula:
**K = A**^{**a**}**+B**^{**b**}**+C**^{**c**}**+D**^{**d**}**+E**^{**e**}
wherein
**A** is acrylamide (AA), methacrylamide (MAA), *N*-[tris(hydroxymethyl)methyl]-acrylamide (TA), or any other monomer based on derivatives of acrylic and methacrylic acids;
**B** is *N,N*'-methylenebisacrylamide (Bis), *N,N'*-1,2-dihydroxyethylenebisacrylamide (DHEBA), polyethyleneglycol diacrylamide (PEGDA), a mixture thereof or any other cross-linking water soluble agent;
**C** is a modified oligonucleotide, modified nucleic acid, protein or other molecule bearing an unsaturated group;
**D** is glycerol, sucrose, *N,N*-dimethylformamide, dimethylsulfoxide, polyhydric alcohols (e.g., polyvinyl alcohol), or other water soluble high-boiling compound;
**E** is water;
**K** is a composition;
**a,b,c,d,e** are percentages (X) of each ingredient in the initial composition (for solids X = m/v×100% and for liquids X = v/v×100%);
3%≤**a**+**b**≤40%; 0.0001%≤**c**≤10%; 0%≤**d**≤90%; 5%≤**e**≤95%.

41. A modified oligonucleotides of the following structure: 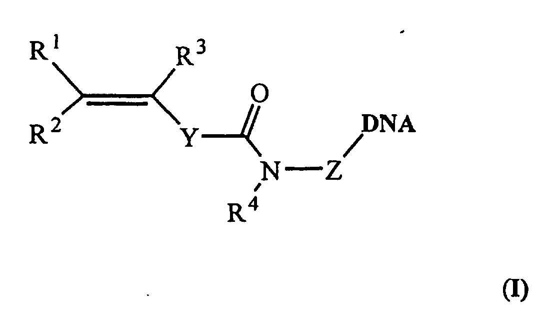 wherein
R¹, R², R³ represent H, alkyl C₁-C₆, Ph, PhCH₂- ;
Z is (CH₂)ₙCH(CH₂OH)CH₂OX where n = 1-6; or (CH₂)ₙ-OX where n = 2-6;
X is a phosphodiester group binding an unsaturated moiety to 5'- and/or 3'-end of the oligonucleotide;
R⁴ represents H, (CH₂)ₙOH where n = 2-6;
Y is (*p*-C₆H₄)ₙ where n = 0-2.

42. Phosphoramidites of general formula: 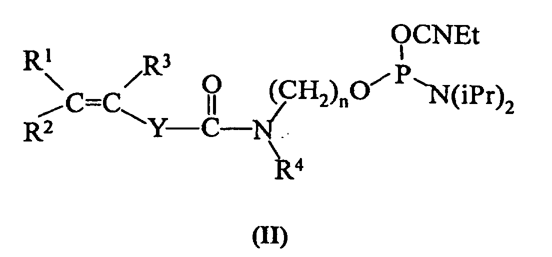 wherein
R¹, R² are H, alkyl C₁-C₆, Ph, PhCH₂-, where n = 2-6;
R³ is alkyl C₁-C₆;
R⁴ represents H, (CH₂)ₙODMT where n = 2-6;
Y is (*p*-C₆H₄)ₙ where n = 0-2.

43. A carrier porous glass (CPG) of the following structure: 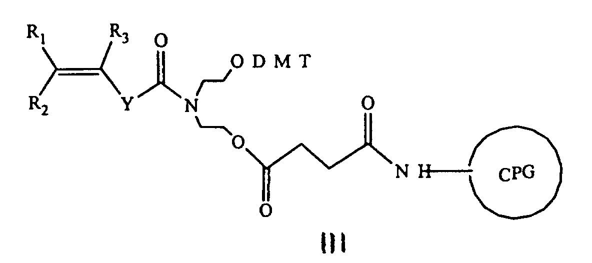 wherein:
R¹, R² are H, alkyl C₁-C₆, Ph, PhCH₂-;
R³ is alkyl C₁-C₆;
Y is (*p*-C₆H₄)ₙ, where n = 0-2.

44. A modified DNA fragments of the following structure: 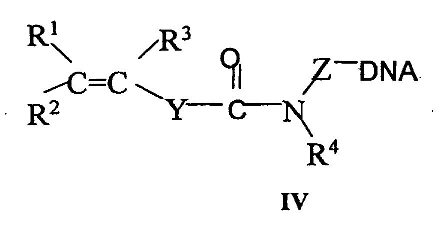 wherein:
R¹, R², R³ are H, alkyl C₁-C₆, Ph, PhCH₂- ;
R⁴ represents H, (CH₂)ₙOH where n = 2-6;
Y is (p-C₆H₄)ₙ where n = 0-2;
Z is (CH₂)ₙCH(CH₂OH)CH₂OX where n = 1-6; or -(CH₂)ₙ-OX where n = 2-6;
X is a phosphodiester group binding an unsaturated moiety to 5'- and/or 3'-end of the oligonucleotide,
or
**5'-X-O-DNA-3'-O-Z (V)**
wherein
X is H, H₂PO₃, -CO-Y-CR³=CR²R¹;
Z represents H, H₂PO₃, -CO-Y-CR³=CR²R¹
R¹, R², R³ are H, alkyl C₁-C₆, Ph, PhCH₂-;
Y represents (*p*-C₆H₄)ₙ where n = 0-2.
or 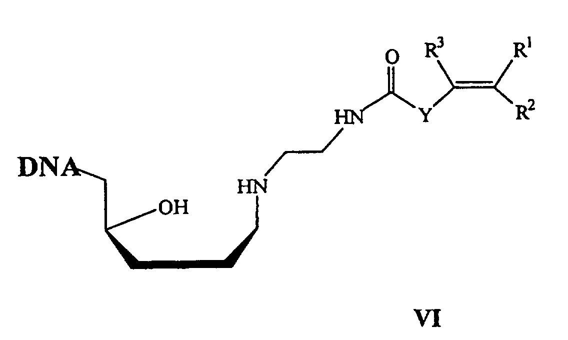 wherein:
R¹, R², R³ are H, alkyl C₁-C₆, Ph, PhCH₂- ;
Y is (p-C₆H₄)ₙ where n = 0-2.

45. A modified proteins of the following structure: 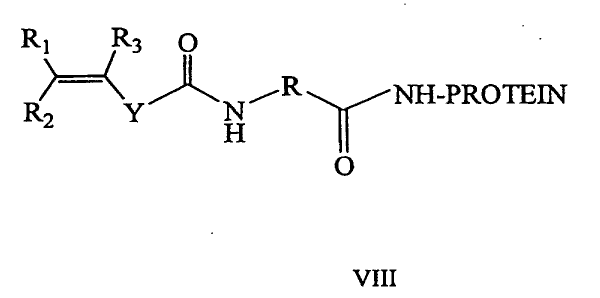 wherein
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, n = 1-20;
Or 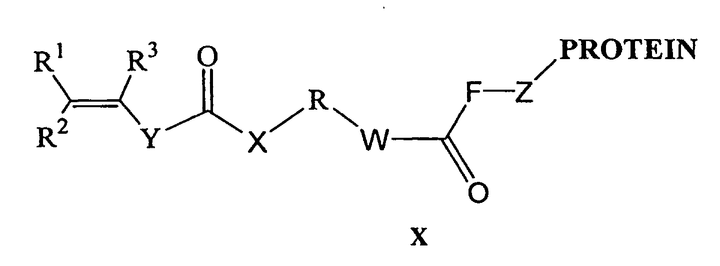 wherein
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, n = 1-20;
X is NH, O, S, CH₂ ;
W is NH, O, CH₂;
F is (CH₂)ₙ, n=1, 2;
Z is NH, S.
or

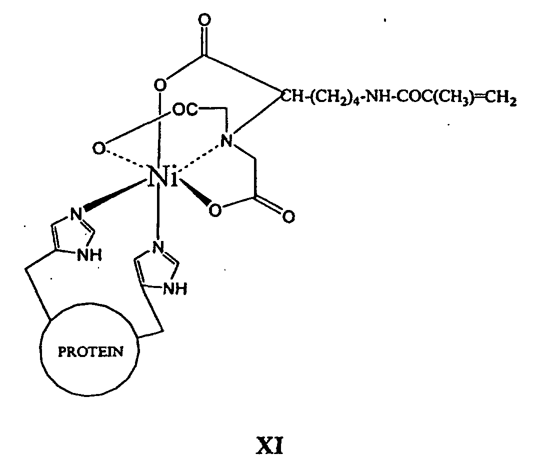

46. α,β-Unsaturated carbonyl compounds of the following structure: 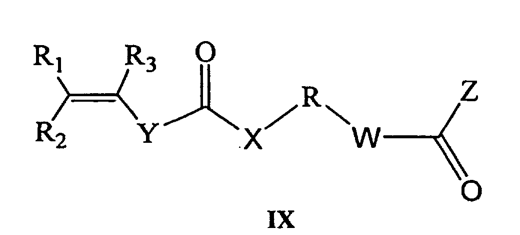 wherein
R¹, R², R³ are H, alkyl C₁-C₆, Ph, PhCH₂-;
Y is (*p*-C₆H₄)ₙ, where n = 0-2;
R is (CH₂)ₙ, (CH₂O)ₙ, n = 1-20;
X is NH, O, S, CH₂;
W is NH, O, CH₂;
Z is a halomethyl, vinyl, succinimidyl, or any other fragment comprising an active multiple bond.

47. Activated esters of the following structure: 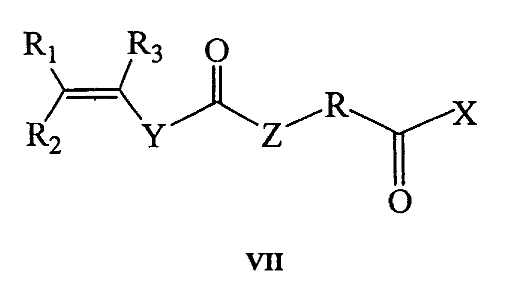 wherein:
R¹, R², R³ are H, alkyl C₁-C₆, Ph, PhCH₂- ;
Y is (*p*-C₆H₄)ₙ, where n = 0-2;
R is (CH₂)ₙ, (CH₂O)ₙ, where n = 1-20;
X is N-hydroxysuccinimidyl-, *p*-nitrophenyloxy-, pentafluoro phenyloxy-, or any other readily leaving acceptor group;
Z is NH, O, CH₂, S.

48. N-Methacryoyl nitrilotriacetic acid of the following structure:
**CH**_{**2**}**=C(Me)-CO-NH-R-CH(COOH)-N(CH**_{**2**}**COOH)**_{**2**}**,**
wherein R represents (CH₂)ₙ, (CH₂CH₂O)ₙ, n = 0-20.

49. A method for performing the PCR-amplification of specific DNA fragments wherein said amplification proceeds both over and inside of plurality of gel cells of microchip being prepared by a copolymerization method comprising the following steps:
(a) preparation of microchip having a specific sets of oligonucleotides (primers) being immobilized by method of copolymerization in certain addresses (three-dimensional isolated gel cells) of the chip;
(b) addition of solution comprising an amplification buffer primers, and nucleic acids under investigation;
(c) carrying out the PCR-amplification by method of thermo cycling treatment to result in forming numerous amplification products and in performing an allele specific elongation a realization of primers immobilized.

50. A method for performing the PCR-amplification of specific DNA fragments wherein said amplification proceeds inside of plurality of microchip's gel cells surrounded with a hydrophobic liquid, which microchip is prepared by a copolymerization method comprising the following steps:
(a) preparation of chip having a specific sets of (primers) being immobilized by method of copolymerization inside each of gel cells;
(b) addition of solution comprising nucleic acids under investigation and nucleic acids under investigation and realization of said nucleic acids hybridization with primers immobilized inside of gel cells;
(c) replacement of hybridization solution with the amplification solution;
(d) replacement of water amplification solution with the hydrophobic liquid (mineral oil) which completely isolates chip cells with each other and surrounds each of cells;
(e) realization of PCR-amplification inside each of gel cells by method of thermo cycling treatment.
